# EUROPÄISCHE PATENTSCHRIFT

(11) **EP 1 606 045 B1**
(45) Veröffentlichungstag und Bekanntmachung des Hinweises auf die Patenterteilung: **01.07.2015**
(21) Anmeldenummer: 04720867.3
(22) Anmeldetag: 16.03.2004
(51) Int. Cl.: B01F 13/02, B65D 65/46

(54) **MISCHVORRICHTUNG**
MIXING DEVICE
DISPOSITIF DE MELANGE

(30) Priorität: 22.03.2003 DE 10312895; 03.03.2004 DE 102004010975
(43) Veröffentlichungstag der Anmeldung: 21.12.2005
(73) Patentinhaber: Henkel AG & Co. KGaA, 40589 Düsseldorf (DE)
(72) Erfinder: HÖFFKES, Horst, 40595 Düsseldorf (DE); BERNECKER, Ullrich, 52074 Aachen (DE); SEILER, Martina, 47228 Duisburg (DE)
(86) Internationale Anmeldenummer: PCT/EP2004/002694
(87) Internationale Veröffentlichungsnummer: WO 2004/082818

(56) Entgegenhaltungen:
- EP-A- 0 479 404
- EP-A- 1 037 589
- EP-A- 1 308 151
- WO-A-95/11861
- DE-A- 4 217 920
- DE-A- 10 059 291
- DE-A- 19 613 941
- DE-A- 19 909 661
- DE-B- 1 053 739
- GB-A- 130 548
- GB-A- 666 244
- GB-A- 2 118 961
- GB-A- 2 356 842
- US-A- 1 332 985
- US-A- 2 592 485
- US-A- 5 063 057
- US-A- 5 116 388
- US-A- 5 270 054
- US-A- 5 484 598
- US-A- 5 788 369
- US-B1- 6 187 058

## Beschreibung

Die Erfindung betrifft ein Mischset mit einer Mischvorrichtung zum Mischen eines in einem von einer Flüssigkeit löslichen Folienbeutel aufgenommenen stückigen, gelartigen, pastösen, pulverförmigen, flüssigen Produktes oder dergleichen mit der Flüssigkeit und ggf. wenigstens einer weiteren Komponente.

Beispielsweise bei pulverförmigen Kosmetikprodukten, die für ihre Anwendung in eine flüssigkeitsbasierte, insbesondere wasserbasierte Form überführt werden müssen, ist darauf zu achten, dass beim Überführen des pulverförmigen Produktes in die Flüssigkeit möglichst keine Staubbelästigung für den Anwender stattfindet. Eine solche Staubbelästigung ist insbesondere dann unerwünscht bzw. zu vermeiden, wenn es sich um stark saure, stark alkalische oder chemisch aktive pulverförmige Produkte handelt, wie z.B. Blondierpulver.

Zur Vermeidung des Staubanfalls sind u.a. Granulationsverfahren vorgeschlagen worden, für Blondierpulver insbesondere die Entstaubung mit Ölkomponenten. Diese Granulationsverfahren mit oxidierbaren Komponenten beinhalten aber neben einer erschwerten Mischbarkeit des Pulvers mit den flüssigen Komponenten vor der Anwendung auch potentielle Risiken, z.B. hinsichtlich einer verringerten Lagerstabilität wegen der gleichzeitigen Anwesenheit oxidierender und oxidierbarer Komponenten. Manchmal ist auch die Formulierung von Komponenten, die der Hydrolyse (oder ggf. einer Solvatolyse in polaren Lösungsmitteln) unterliegen können, in ölbasierten Rezepturen von Interesse.

Es ist bereits vorgeschlagen worden, Blondierpulver in einem wasserlöslichen Folienbeutel zu verpacken. Das Pulver wird dann im Folienbeutel in eine Flüssigkeit gegeben, der Folienbeutel löst sich nach und nach auf und die Mischung des Pulvers mit der Flüssigkeit kann erfolgen. In der Praxis hat sich jedoch die verzögerte Löslichkeit des Folienbeutels als nachteilig herausgestellt, d.h. die Zubereitungszeit ist zu lang und für den Anwender nicht akzeptabel.

Aufgabe der Erfindung ist es deshalb, die Nachteile des Standes der Technik zu überwinden. Diese Aufgabe wird durch ein Mischset mit den Merkmalen von Anspruch 1 gelöst. Durch einen verschließbaren Mischbehälter mit einem Aufnahmeraum für das in dem Folienbeutel aufgenommene Produkt und die Flüssigkeit und die ggf. weiteren Komponenten, wobei im Bereich des Aufnahmeraumes Einbauten zur mechanischen Zerstörung des Folienbeutels vorgesehen sind und einen Folienbeutel, bei dem mindestens eine Oberfläche eine dreidimensionale Struktur, insbesondere eine aufgeprägte dreidimensionale Struktur aufweist. Hierbei ist der Mischmechanismus, für sämtliche Aggregatzustände des Produktes, insbesondere stückig, gelartig, pastös, pulverförmig, flüssigen oder dergleichen, verwendbar.

Mit einer solchen Mischvorrichtung ist es möglich, nach dem Einfüllen der Komponenten und Verschließen des Mischbehälters den das pulverförmige Produkt aufnehmenden Folienbeutel mechanisch zu zerstören bzw. zu zerkleinern, was zu einer beschleunigten Auflösung der Folie und einer deutlich verkürzten Mischzeit des Produktes mit den restlichen Komponenten führt. Dabei können die Einbauten im Aufnahmeraum des Mischbehälters grundsätzlich unterschiedlich gestaltet sein, wobei bei den konstruktiv einfachsten Ausgestaltungen ein Wirksamwerden der Einbauten dadurch auf einfache Weise erreicht werden kann, dass die Mischvorrichtung vom Anwender geschüttelt wird, was ohnehin sinnvoll bzw. zweckmäßig ist, um den Mischvorgang an sich zu beschleunigen.

Um die Handhabung der Vorrichtung beim Öffnen und Verschließen zu erleichtern, ist vorteilhaft vorgesehen, dass der Mischbehälter eine von einem abnehmbaren Deckel verschließbare Behälteröffnung aufweist. Die zu mischenden Komponenten können dann nach Abnehmen des Deckels einfach in den Aufnahmeraum des Mischbehälters eingefüllt werden, anschließend kann der Deckel wieder aufgesetzt werden.

Besonders zweckmäßig ist es, wenn die Einbauten als im Bereich der Behälteröffnung angeordneter Einsatz ausgebildet sind. Nach dem Mischvorgang und dem Öffnen des Behälterdeckels kann dann der Einsatz aus dem Bereich der Behälteröffnung entnommen werden und das nach dem Mischen fertige Produkt läßt sich problemlos aus dem Behälter entnehmen.

Nach einer ersten vorteilhaften Ausgestaltung ist vorgesehen, dass der Einsatz zum Aufnahmeraum hin zitruspressenartig ausgebildet ist.

Alternativ kann auch vorgesehen sein, dass der Einsatz siebplattenartig mit in den Aufnahmeraum gerichteten, spitz zulaufenden Stiften oder Dornen ausgebildet ist. Des weiteren kann auch vorgesehen sein, den Einsatz mit messerförmigen nach innen gerichteten Elementen auszustatten.
Zusätzlich können sämtliche Kombinationen aus den erwähnten Einsätzen oder Weiteren vorgesehen sein.
Der Folienbeutel besteht vorteilhaft aus Polyvinylalkohol oder Gelatine, allgemein jedoch aus einem, durch die zu mischende Zugabeflüssigkeit, löslichen Feststoff.

In vorteilhafter Ausgestaltung ist vorgesehen, dass das Produkt ein Blondierpulver und die Flüssigkeit eine Wasserstoffperoxidlösung ist. Die Mischvorrichtung kann dann zum Herstellen eines Blondiermittels eingesetzt werden. Dabei ist dann vorteilhaft die weitere Komponente eine Blondiercreme.

### Kosmetische Portion

In einer vorteilhaften Ausführungsform der vorliegenden Erfindung beinhaltet das erfindungsgemäße Mischset eine kosmetische Portion. Diese Portion besteht aus einer von einer Flüssigkeit löslichen Umhüllung, insbesondere einem entsprechenden Folienbeutel, und einem darin aufgenommenen Produkt.

Die gestellte Aufgabe wird bei dieser erfindungsgemäßen Ausführungsform durch die mechanische Einwirkung in der Mischvorrichtung und durch die konkrete Gestaltung des Folienbeutels gelöst.

Auf dem Gebiet der Kosmetik besteht ein großer Bedarf an Produkten die einerseits effektiv wirksam sein sollen und andererseits für den Verbraucher einfach und vor allem gefahrlos handhabbar und anwendbar sein sollen. Insbesondere auf dem Gebiet der Haarkosmetik haben sich in den vergangenen Jahren Blondier- und Haarfärbesysteme entwickelt, die äußerst effektiv wirken, jedoch bei unsachgemäßer Handhabung, beispielsweise bei Kontaminierung mit Hautpartien oder Augen zu Irritationen oder in Extremfällen sogar zum Auslösen von Allergien führen können. Es bestand daher ein großer Bedarf, die Sicherheit der Handhabung solcher kosmetischer Formulierungen zu gewährleisten und darüber hinaus dem Verbraucher ein einfach zu dosierendes Verpackungssystem an die Hand zu geben, das auch ein Anmischen bzw. eine Zusammenstellung der benötigten Komponenten vor Ort beim Verbraucher ermöglicht.

Im Stand der Technik werden in wasserlösliche Beutel-verpackte Haarkosmetikformulierungen bereits offenbart. DE 196 13 941 A1 beschreibt ein Verfahren zur Herstellung von nichtstaubenden pulverförmigen Mitteln zum Blondieren von menschlichen Haaren. Die Blondiermittel weisen mindestens eine Peroxidverbindung auf, die mit geeigneten Verdickungsmitteln versetzt und anschließend für den Transport und die Weiterverarbeitung portionsweise in wasserlösliche Beutel verpackt werden.

EP-A1-1037589 offenbart ein Mittel zur Behandlung keratinischer Fasern, bestehend aus mindestens einer wässerigen Zubereitung A und mindestens einer davon räumlich getrennt vorliegenden Zubereitung B, die einen in der Zubereitung A nicht lagerstabilen Bestandteil, ausgewählt aus der Gruppe, die von Parfümölen sowie Vitaminen, Provitaminen und deren Derivaten gebildet wird, enthält, wobei der Folienbeutel mit der Zubereitung B aus einem Material, das bei Zugabe der Zubereitung B zu einer Zubereitung A eine Vermischung der Komponenten beider Zubereitungen bei 38 °C innerhalb von 5 Minuten ermöglicht.

US 5,116,388 offenbart Haarfärbemittel auf Basis von Oxidationsfarbstoffen sowie Bleichmittel zum Blondieren von Haaren, die in Polyvinylalkohol-Verpackungen eingearbeitet werden, um die durch Pulverstaub verursachten Irritationen zu vermindern.

Die im Stand der Technik offenbarten portionierten kosmetischen Formulierungen bieten zwar eine verbesserte Handhabbarkeit und eine Verringerung der Staubkontamination der verpackten kosmetischen Zubereitungen, jedoch weisen die in wasserlöslichen Foliensystemen verpackten Portionen den Nachteil auf, dass sie sich in Wasser nur langsam lösen. Darüber hinaus weisen die im Stand der Technik offenbarten wasserlöslichen Kosmetikportionen, insbesondere auf dem Gebiet der Haarkosmetik, den Nachteil auf, dass die Portionen den Verbrauchern aus den Händen gleiten können und gegebenenfalls dabei zerbersten. Nicht selten haben Verbraucher, die haarkosmetische Produkte anwenden, feuchte Hände bzw. Finger, beispielsweise deshalb, weil die Haare kurz vor der Anwendung gewaschen wurden, was die Gefahr des Abrutschens der Kosmetikportionen extrem erhöht. Viele der im Stand der Technik offenbarten kosmetischen Einzelportionen werden häufig in einer weiteren wasserundurchlässigen Umverpackung in den Handel gebracht. Die Umverpackungen bestehen dabei häufig aus glatten Folienbeuteln oder metallbeschichteten glatten Verpackungssystemen, so dass die im Stand der Technik beschriebenen wasserlöslichen PVA-Kosmetikportionen plan auf den Oberflächen der Umverpackungsmaterialien anliegen und durch hohe Adhäsionskräfte nicht ohne Weiteres aus dem Umverpackungsbehältnis gleiten. Aufgabe der erfindungsgemäßen Ausführungsform ist es, portionierte kosmetische Zubereitung in wasserlöslichen und/oder wasserdispergierbaren Folienbeuteln bereitzustellen, die die vorgenannten Probleme des Standes der Technik nicht aufweisen.

Es wurde gefunden, dass die vorgenannten Probleme durch die spezielle Ausgestaltung einer Portion gelöst werden.

Eine Portion umfassend eine kosmetische Zubereitung und einen wasserlöslichen und/oder wasserdispergierbaren Folienbeutel, wobei dieser Folienbeutel die kosmetische Zubereitung umhüllt und die Oberfläche des Folienbeutels einen quadratischen Mittelwert der Rauheit von wenigstens 10 µm aufweist.

Diese Portionen weisen kosmetische Zubereitungen auf, die von wasserlöslichen und/oder wasserdispergierbaren Folienbeuteln umhüllt sind. Vorteilhaft sind jedoch in Wasser vollständig lösliche Folienbeutel. Im Rahmen der vorliegenden erfindungsgemäßen Ausführungsform wird der Begriff "Portion" oder "Kosmetikportion" synonym mit dem Begriff "portionierte kosmetische Zubereitung in wasserlöslichem und/oder wasserdispergierbarem Folienbeutel" verwandt. Die Portionen der erfindungsgemäßen Ausführungsform weisen einen Folienbeutel auf, dessen Oberfläche vorteilhaft einen quadratischen Mittelwert der Rauheit von wenigstens 10 µm aufweist. Vorzugsweise zeigt die Oberfläche des Folienbeutels einen quadratischen Mittelwert der Rauheit von 10 bis 100 µm, besonders vorteilhaft von 10 bis 50 µm und insbesondere von 30 bis 35 µm auf. Der Begriff "Oberfläche" bezeichnet im Rahmen dieser Ausführungsform die flächigen Bereiche des Folienbeutels, beispielsweise einer Polymerfolie.

Die Bestimmung des quadratischen Mittelwerts der Rauheit des Folienbeutels erfolgte gemäß DIN 4762/1 mit handelsüblichen Oberflächenabtastgeräten.

Kommerziell erhältlich sind Folienbeutelmaterialien auf Basis von Polyvinylalkohol, beispielsweise als Polymerfolien, die die oben angegebenen Rauheitswerte aufweisen, von der Firma Syntana unter dem Markennamen Solublon^{®} PVAL-Folie, Typ SA 20.

Dadurch, dass die erfindungsgemäß einzusetzenden Folienbeutel im Vergleich zu den im Stand der Technik auf diesem Gebiet eingesetzten Folienbeuteln eine wesentlich rauere Oberfläche aufweisen, vergrößert sich auch die dreidimensionale makroskopische Oberfläche des Folienbeutels. Die dreidimensionale makroskopische Oberfläche berücksichtigt zusätzlich die Flächen, die sich durch Unregelmäßigkeiten auf der Folienoberfläche aufspannen. Für den Fall einer ideal glatten Oberfläche entspricht die dreidimensional makroskopische Oberfläche der zweidimensionalen geometrischen Oberfläche. In einer weiteren Ausführungsform ist die dreidimensionale makroskopische Oberfläche des Folienbeutels der Portion mindestens 10 %, vorteilhaft mindestens 20 %, weiter vorteilhaft zwischen 20 und 100 %, äußerst vorteilhaft zwischen 30 und 50 % größer als die zweidimensionale geometrische Oberfläche.

Gegenstand der vorliegenden erfindungsgemäßen Ausführungsform sind somit Portionen umfassend eine kosmetische Zubereitung und einen wasserlöslichen und/oder wasserdispergierbaren Folienbeutel, wobei der Folienbeutel die kosmetische Zubereitung umhüllt und die dreidimensionale makroskopische Oberfläche des Folienbeutels der Portion mindestens 10 %, vorteilhaft mindestens 20 %, weiter vorteilhaft zwischen 20 und 100 %, äußerst vorteilhaft zwischen 30 und 50 % größer als die zweidimensionale geometrische Oberfläche ist.
Die Bestimmung der dreidimensionalen Oberfläche wird ausgehend von der Bezugsoberfläche bestimmt, die die Form der geometrischen Oberfläche hat und in ihrer Lage im Raum mit der Hauptrichtung der wirklichen Oberfläche übereinstimmt.

Die im Vergleich zur zweidimensionalen geometrischen Oberfläche vergrößerte dreidimensionale makroskopische Oberfläche trägt unter anderem zu einer verbesserten Wasserlöslichkeit der erfindungsgemäßen Portionen bei.
Bei den erfindungsgemäßen Portionen ist es vorteilhaft, dass mindestens eine Oberfläche des Folienbeutels eine dreidimensionale Struktur, vorzugsweise eine aufgeprägte dreidimensionale Struktur, aufweist.

Die Außen- und/oder Innenfläche des Folienbeutels kann dabei zu mindestens 50 %, vorzugsweise mindestens 70 %, weiter vorteilhaft mindestens zu 90 % und insbesondere im Wesentlichen vollständig mit einer dreidimensionalen, vorzugsweise aufgeprägten, Struktur versehen sein.

Im Rahmen der vorliegenden erfindungsgemäßen Ausführungsform bezeichnet die Innenfläche des Folienbeutels den flächigen Bereich, der mit der kosmetischen Zubereitung in Kontakt treten kann. Bei Vorliegen eines Folienbeutels ist somit die Oberfläche der Folie im Beutelinneren die Innenfläche und die Folienoberfläche außerhalb des Beutelinneren die Außenfläche. Die Außenfläche steht nicht mit der kosmetischen Zubereitung der erfindungsgemäßen Portion in Kontakt.

Bei der auf einer Oberfläche des Folienbeutels aufgeprägten Struktur handelt es sich in einer vorteilhaften Ausführungsform um eine regelmäßige aufgeprägte dreidimensionale Struktur in Form eines Musters.

Die aufgeprägte Struktur weist auf der Oberfläche in einer vorteilhaften Ausführungsform ein regelmäßiges dreidimensionales Muster auf. Das regelmäßige Muster kann dabei jegliche erdenkliche Form aufweisen, beispielsweise Karos, Rauten, eingestanzte Zylinder, Ovale, etc. In einer vorteilhaften Ausführungsform besteht das regelmäßige Muster in einer periodisch wiederkehrenden Anordnung von Erhöhungen und Vertiefungen der Folienbeuteloberfläche.

Das aufgeprägte Muster kann dabei sowohl die haptischen Eigenschaften der erfindungsgemäßen Portion als auch dessen Auflösegeschwindigkeit beeinflussen. Es hat sich daher als vorteilhaft erwiesen, dass das aufgeprägte Muster mindestens 4, vorzugsweise mindestens 6, besonders vorteilhaft zwischen 8 und 50, weiter vorteilhaft zwischen 10 und 25 Vertiefungen oder Erhöhungen pro 1 cm² der zweidimensionalen Oberfläche aufweist. Aufgeprägte Vertiefungen oder Erhöhungen weisen im Rahmen der vorliegenden erfindungsgemäßen Ausführungsform in ihrer größten Ausdehnung mindestens einen Durchmesser von 2 µm und eine Tiefe bzw. Höhe von mindestens 2 µm vorzugsweise mindestens 5 µm auf.

In einer weiteren vorteilhaften Ausführungsform weist die Oberfläche des Folienbeutels kreisförmige und/oder dreieckige und/oder viereckige und/oder mehreckige Vertiefungen auf.

Die Oberflächen des Folienbeutels können aber auch in einer weiteren Ausführungsform quaderförmige, runde, eckige, ovale, sägezahnförmige oder dreieckig spitz zur Oberfläche laufende Erhöhungen aufweisen.

In einer vorteilhaften Ausführungsform weist die Oberfläche des Folienbeutels ein gitterförmiges oder wabenförmiges dreidimensionales strukturiertes Muster auf. Diese Muster sind vorzugsweise aufgeprägt oder auf die Hülloberfläche aufgestanzt und verleihen somit der Oberfläche ein dreidimensionales Profil. In einer vorteilhaften Ausführungsform weist der Folienbeutel durch Aufprägen eines gitterförmigen oderwabenförmigen Musters Gitternetzlinien auf. Vorteilhaft werden die Gitternetzlinien durch eine Aneinanderreihung von die Vertiefungen begrenzenden Rändern gebildet.

Die vorteilhaften gitterförmigen Muster werden vorzugsweise derart auf die Oberflächen des Folienbeutels aufgeprägt, so dass das Verhältnis der durchschnittlichen Breite von Gitternetzlinie zur maximalen Ausdehnung der Ebene der Vertiefung kleiner 20:1, vorzugsweise kleiner 10:1, besonders vorteilhaft kleiner 1:1, weiter vorteilhaft kleiner 0,5:1 und insbesondere kleiner 0,25:1 ist.

Insbesondere bei aufgeprägten Mustern hat es sich als vorteilhaft herausgestellt, dass das Verhältnis von durchschnittlichem Durchmesser der Vertiefung zur Tiefe der Vertiefung unterhalb 20:1, vorzugsweise 10:1 bis 1:10, insbesondere 8:1 bis 1:1, im Speziellen 6:1 bis 4:1 beträgt.

Die erfindungsgemäßen Portionen können Folienbeutel aufweisen, die nur einseitig ein aufgeprägtes dreidimensionales Muster aufweisen, insbesondere nur auf der nicht mit der kosmetischen Zubereitung in Kontakt stehenden Außenseite der Hülloberfläche. Vorteilhaft ist es jedoch, dass die Folienbeutel beidseitig ein aufgeprägtes dreidimensionale strukturiertes Muster aufweisen, d.h., dass sowohl die Innen- als auch die Außenseite des Folienbeutels dieses Muster trägt.

Vorzugsweise weisen die erfindungsgemäßen Portionen Folienbeutel auf, deren mittlere Stärke 10 bis 100 µm, vorzugsweise 15 bis 50 µm und insbesondere 20 bis 40 µm beträgt. Die gewählten Folienbeutelstärken tragen insbesondere, wenn es sich bei den Folienbeuteln um wasserlösliche und/oderwasserdispergierbare Folien handelt, zu einer optimalen Wasserauflösegeschwindigkeit und zudem zu einer guten Verarbeitbarkeit der Folien bei. So hat sich gezeigt, dass insbesondere in dem Bereich von einer mittleren Folienstärken zwischen 10 und 100 µm das thermische Versiegeln, insbesondere flüssigkeitsdichtes Versiegeln, problemlos durchgeführt werden kann. Die Folienstärke kann sich dabei auf Teilbereiche oder vorteilhaft auf das gesamte Hüllmaterial beziehen. Die mittlere Folienstärke bezieht sich auf das Querschnittsprofil und wurde über die Erhöhungen und Vertiefungen entlang einer 1 cm langen Profilstrecke gemittelt. Ein 1 Quadratzentimeter großes Folienstück (1 cm x 1 cm) des Hüllmaterials wird in 5 äquidistante Streifen (jeweils 2 mm) zerlegt und jeweils die mittlere Folienstärke entlang des Profils ermittelt. Der Mittelwert aus den 5 Messungen bildet die mittlere Folienstärke. Die Bestimmung der mittleren Folienstärke entlang des Querschnittprofils mittels Videolichtmikroskopie bestimmt.

Das Material der wasserlöslichen und/oder wasserdispergierbaren Folienbeutel der erfindungsgemäßen Portionen besteht in einer vorteilhaften Ausführungsform ganz oder teilweise aus einem Thermoplast, ausgewählt aus der Gruppe umfassend Polyvinylalkohol (PVA), acetalisierter Polyvinylalkohol, Polyvinylpyrrolidon, Polyethylenoxid, Gelatine, Cellulose, Stärke und Derivate der vorgenannten Stoffe und/oder Mischungen der vorgenannten Polymere, wobei Polyvinylalkohol besonders vorteilhaft ist.

Die vorstehend beschriebenen Polyvinylalkohole sind kommerziell verfügbar, beispielsweise unter dem Warenzeichen Mowiol^{®} (Clariant). Im Rahmen der vorliegenden erfindungsgemäßen Ausführungsform besonders geeignete Polyvinylalkohole sind beispielsweise Mowiol^{®} 3-83, Mowiol^{®} 4-88, Mowiol^{®} 5-88, Mowiol^{®} 8- 88 sowie Clariant L648.

Weitere als Material für den Folienbeutel geeignete Polyvinylalkohole sind ELVANOL^{®} 51-05, 52-22, 50-42, 85-82, 75-15, T-25, T-66, 90-50 (Warenzeichen der Du Pont), ALCOTEX^{®} 72.5, 78, B72, F80/40, F88/4, F88/26, F88/40, F88/47 (Warenzeichen der Harlow Chemical Co.), Gohsenol^{®} NK-05, A-300, AH-22, C-500, GH-20, GL-03, GM-14L, KA-20, KA-500, KH-20, KP-06, N- 300, NH-26, NM11Q, KZ-06 (Warenzeichen der Nippon Gohsei K. K.).

In einer weiteren vorteilhaften Ausführungsform weist das Folienbeutelmaterial zusätzlich Polymere ausgewählt aus der Gruppe, umfassend Acrylsäure-haltige Polymere, Polyacrylamide, Oxazolin-Polymere, Polystyrolsulfonate, Polyurethane, Polyester, Polyether und/oder Mischungen der vorstehenden Polymere, auf. Vorteilhaft ist, wenn das Hüllmaterial der erfindungsgemäßen Portion ein teilacetalisierter Polyvinylalkohol mit einem Verseifungsgrad von 70 bis 100 Mol-%, vorzugsweise 80 bis 96 Mol-%, besonders vorteilhaft 82 bis 94 Mol-% und insbesondere 85 bis 89 Mol-% ausmacht. Weiter vorteilhaft ist, dass der verwendete wasserlösliche Thermoplast ein Polyvinylacetat umfasst, dessen mittleres Molekulargewicht im Bereich von 10000 bis 100000 gmol⁻¹, vorzugsweise von 11000 bis 90000 gmol⁻¹, besonders vorteilhaft von 12000 bis 80000 gmol⁻¹, insbesondere von 13000 bis 70000 gmol⁻¹ und im Speziellen von 20000 bis 40000 gmol⁻¹ ist. Die mittleren Molekulargewichte wurden mittels Gelpermeationschromatographie bestimmt. Es wurde überraschend gefunden, dass über die spezielle Auswahl des Molekulargewichts die Auflösegeschwindigkeit erheblich verbessert werden kann.

In einer weiteren Ausführungsform, umfasst das Hüllmaterial die genannten Thermoplasten in Mengen von mindestens 50 Gew.-%, vorzugsweise von mindestens 70 Gew.-%, besonders vorteilhaft von mindestens 80 Gew.-% und insbesondere von mindestens 90 Gew.-%, jeweils bezogen auf das Gewicht des gesamten Hüllmaterials.

Die erfindungsgemäßen Portionen weisen in einer weiteren vorteilhaften Ausführungsform der vorliegenden Erfindung ein Innenvolumen von 5 bis 500 cm³, vorzugsweise von 10 bis 200 cm³, besonders vorteilhaft von 20 bis 100 cm³ und insbesondere von 30 bis 70 cm³ auf. Insbesondere auf dem Gebiet der Haarbehandlungsmittel haben sich Portionsgrößen mit einem Innenvolumen von 30 bis 70 cm³ als besonders geeignet herausgestellt, da sie einerseits für den Endverbraucher einfach zu handhaben sind und andererseits durch das begrenzte Innenvolumen keine Probleme bezüglich der durch die Schwerkraft der kosmetischen Zubereitung bedingte Schäden an dem Folienbeutel auftreten.

Das Innenvolumen der Portion bezeichnet den Raum der Portion, der befähigt ist, die kosmetische Zubereitung aufzunehmen.

In einer weiteren Ausführungsform der vorliegenden Erfindung löst sich der Folienbeutel in Wasser bei 20 °C innerhalb von weniger als 5 Minuten, vorzugsweise weniger als 4 Minuten und weiter vorteilhaft weniger als 3 Minuten und insbesondere zwischen 2 und 0,5 Minuten in Wasser auf. Die Bestimmung der Auflösegeschwindigkeit erfolgte durch Zugabe von 0,07 g Hüllmaterial in ein Becherglas mit 7 ml Wasser. Das Wasser wurde während derAuflösungsphase mit Hilfe eines Magnetrührers gerührt (60 U/min). Die Auflösegeschwindigkeit wurde mittels optischer Methoden bestimmt, beginnend vom Zugabezeitpunkt des Hüllmaterials zur gerührten wässrigen Lösung bis zur vollständigen Auflösung (Trübungsmessung).

Einzeldosierungen werden von dem Verbraucher als bequem empfunden. Der Verbraucher nimmt das entsprechende Produkt, dosiert es und braucht sich über die Abmessung geeigneter Mengen keine Gedanken zu machen. Es kann jedoch Situationen geben, in denen diese Angebotsformen kritisch sind, da eine situationsabhängige Abänderung der Dosierung nicht mehr möglich ist und somit beispielsweise mit einer Dosiereinheit zu wenig, mit zwei Einheiten aber zuviel dosiert wird. Dieses Problem kann gelöst werden, in dem Mehrkammerfolienbeutel zur Verfügung gestellt werden. Ein weiterer Gegenstand der vorliegenden erfindungsgemäßen Ausführungsform ist daher ein Mehrkammerfolienbeutel, bestehend aus mindestens einer erfindungsgemäßen Portion.

In einer weiteren vorteilhaften Ausführungsform besteht der Mehrkammerfolienbeutel aus zwei oder mehrstückigen Einzelportionen, die über Stege miteinander verbunden sind. Stege können dabei auch gemeinsame Siegelflächen zweier benachbarten Portionen sein.

In einer weiteren vorteilhaften Ausführungsform liegen die Mehrkammerfolienbeutel derart vor, dass sie zusammenhängend zwei oder drei Einzelportionen aufweisen, die vorteilhaft jeweils unterschiedliche kosmetische Zubereitungen aufweisen. Ein Mehrkammersystem im Sinne der vorliegenden erfindungsgemäßen Ausführungsform kann somit auch ein System sein, beispielsweise zweier Farbstoffkomponenten (Entwickler und Kuppler), die sich in einem Mehrkammerfolienbeutel getrennt voneinander befinden. Vorteilhaft weisen die erfindungsgemäßen Mehrkammerfolienbeutel zwei oder mehr Kompartimente auf, in denen sich vorzugsweise unterschiedliche kosmetische Zubereitungen, insbesondere Haarfärbemittelkomponenten befinden. Solche Mehrkammerfolienbeutel vereinfachen die Dosierung für den Verbraucher weiter, da in einem solchen Fall lediglich eine einzige Verpackungseinheit in der entsprechenden Anwendungslösung aufgelöst werden muss.

Die Oberfläche des Folienbeutels erhält eine aufgeprägte Struktur durch Erhitzen und Pressen, vorzugsweise vor dem Befüllen des Folienbeutels mit einer kosmetischen Zubereitung.

Vorzugsweise handelt es sich bei dem Hüllmaterial um Blas- oder insbesondere Gießpolymerfolien, die im erwärmten Zustand mit einer Matrize geprägt werden, um ihnen die vorteilhaften Oberflächeneigenschaften zu verleihen. Vorzugsweise erfolgt der Prägevorgang derart, dass die Folie einseitig mit dem Matrizenwerkzeug gepresst wird, so dass einseitig eine dreidimensionale Struktur aufgeprägt wird, die sich auf der gegenüberliegenden Seite als "negativ" wiederfindet.

Üblicherweise werden die erfindungsgemäßen Portionen in einer Verkaufseinheit (Kit) mit einem Mischset und gegebenenfalls einer oder mehreren weiteren Zubereitungen und/oder Kosmetikapplikationsvorrichtungen vermarktet. Ein weiterer Gegenstand der vorliegenden erfindungsgemäßen Ausführungsform ist daher ein Kit enthaltend ein Mischset und ein oder mehrere erfindungsgemäße Portionen.

In einer weiteren vorteilhaften Ausführungsform enthält das erfindungsgemäße Kit zusätzlich ein oder mehrere Bestandteile ausgewählt aus der Gruppe
a) ein oder mehrere weitere Aufnahmebehältnis(se) enthaltend mindestens eine weitere kosmetische Zubereitung, vorzugsweise eine WasserstoffperoxidLösung oder Wasserstoffperoxid-Emulsion oder eine Pflegelotion; und/oder
b) ein oder mehrere Sicherheitsmaterialien zur Vermeidung des unerwünschten Inkontakttretens der kosmetischen Zubereitung mit dem menschlichen Körper, vorzugsweise Handschuhe.

In einer besonders vorteilhaften Ausführungsform enthält das erfindungsgemäße Mischset mindestens eine erfindungsgemäße Portion mit einem Blondierpulver als Zubereitung, eine Mischvorrichtung, sowie vorzugsweise eine Kunststoffflasche mit einer wässrigen Wasserstoffperoxidlösung oder Wasserstoffperoxidemulsion oder Wasserstoffperioxdispersion und ggf. zusätzlich eine Pflegelotion.

In einer weiteren vorteilhaften Ausführungsform enthält das erfindungsgemäße Kit mindestens ein oder mehrere erfindungsgemäße Portionen, die als kosmetische Zubereitung Blondierpulver enthalten, ein oder mehrere Behältnisse, enthaltend eine Wasserstoffperoxidemulsion oder Wasserstoffperoxiddispersion, eine Mischvorrichtung zum Vermischen der Komponenten, und Sicherheitshandschuhe sowie ggf. eine oder mehrere Strähnenhauben oder Strähnennadeln.

In einer weiteren vorteilhaften Ausführungsform der Erfindung enthält das erfindungsgemäße Kit mindestens ein oder mehrere erfindungsgemäße Portionen enthaltend ein Haarfärbemittel, vorzugsweise eine Haarfärbemittelvorstufe, besonders vorteilhaft die Komponente A eines Oxofärbemittels. Weiterhin enthält das Kit eine Mischvorrichtung zum Vermischen der Komponenten und vorzugsweise zusätzlich ein oder mehrere Portionen enthaltend eine weitere Haarfärbekomponente, vorzugsweise die Komponente B eines Oxofärbemittels sowie ggf. zusätzlich eine Oxidationsmittelzubereitung (C), die entweder wässrig, z.B. im Falle von Wasserstoffperoxid sein kann oder auch in Form in eines wasserfreien Pulvers. z.B. eines Wasserstoffperoxidadukts an Harnstoff (Percarbamid oder an Melamin (Melaminperhydrat) oder in Form einer anderen Perverbindung, z.B. Magnesiumperoxid oder Kaliumpersulfat vorliegen kann.

Ein weiterer Gegenstand ist die Verwendung der zuvor beschriebenen wasserlöslichen und/oder wasserdispergierbaren Folienbeutel zur Portionierung kosmetischer Zubereitungen. Die Oberfläche des Folienbeutels weist, wie zuvor beschrieben, einen quadratischen Mittelwert der Rauheit von wenigstens 10 µm auf. Unter Portionierung ist, im Rahmen der vorliegenden erfindungsgemäßen Ausführungsform, das Einteilen von stofflichen Mengen in geeignete handhabbare Größen zu verstehen, z.B., die für den einmaligen Haarfärbe- oder Haarblondiervorgang benötigte Menge. Diese Mengeneinheiten werden mittels des Hüllmaterials zu Portionen verpackt, z.B. zu wasserlöslichen Beuteln oder Kapseln.

Ein weiterer Gegenstand ist die Verwendung eines wasserlöslichen und/oder wasserdispergierbaren Folienbeutels zur Portionierung kosmetischer Zubereitungen, die vorzugsweise die zuvor beschriebenen Rauheitswerte aufweisen. Die Verwendung erfolgt im Rahmen dieses Gegenstandes mit einer Umhüllung, die eine dreidimensionale makroskopische Oberfläche aufweist, die mindestens 10 %, vorteilhaft mindestens 20 %, weiter vorteilhaft mindestens 30 % und 50 % größer als die zweidimensionale geometrische Oberfläche ist.

Die Erfindung ist nachstehend anhand der Zeichnung und Ausführungsbeispiele beispielhaft näher erläutert. Diese zeigt jeweils in einem Längsschnitt in:
- Fig. 1: eine Mischvorrichtung nach einer ersten Ausgestaltung und
- Fig. 2: eine Mischvorrichtung nach einer zweiten Ausgestaltung.

Eine erfindungsgemäße Mischvorrichtung ist in der Zeichnung allgemein mit 1 bezeichnet. Diese Mischvorrichtung 1 weist einen Mischbehälter 2 auf, der oberseitig mit einer Behälteröffnung 3 versehen ist, welche mit einem Behälterdeckel 4 verschließbar ist, wobei beim Ausführungsbeispiel der Behälterdeckel 4 aufschraubbar ist, ein Schraubgewinde ist mit 5 bezeichnet.

Alternativ zu der Schraubverbindung kann auch eine Steckverbindung oder eine Schnapp-Rastverbindung vorgesehen sein.

Im Bereich der Behälteröffnung 3 ist auf den Behälter 2 ein Einsatz 6 aufgesetzt, der zitruspressenartig nach innen zum Aufnahmeraum 7 des Behälters hin ausgewölbt ist. Dieser Einsatz 6 kann von der Behälteröffnung abgenommen werden, um den Aufnahmeraum des Behälters 7 zu füllen, anschließend kann er wieder aufgesetzt werden und verbleibt dann in fest positionierter Lage, nachdem der Behälterdeckel 4 aufgeschraubt worden ist.

Die so ausgebildete Mischvorrichtung 1 dient zur Aufnahme eines in einem von einer Flüssigkeit löslichen Folienbeutel 8 aufgenommenen Produktes 9, bei dem es sich vorzugsweise um ein Blondierpulver handelt.

Neben dem mit dem Produkt 9 gefüllten Folienbeutel 8 wird in den Aufnahmeraum 7 bei geöffnetem Mischbehälter 2 eine Flüssigkeit eingegeben, die in der Lage ist, den Folienbeutel 8 aufzulösen. Bei dieser Flüssigkeit handelt es sich beispielsweise um eine Wasserstoffperoxidlösung. Diese wird aus einem nicht dargestellten Aufnahmebehältnis in den Aufnahmeraum 7 des Mischbehälters 2 eingefüllt. Darüber hinaus kann zusätzlich auch noch eine weitere Komponente, beispielsweise eine Blondiercreme, aus einem ebenfalls nicht dargestellten weiteren Aufnahmebehältnis in den Aufnahmeraum 7 eingegeben werden.

Anschließend wird der zitruspressenartige Einsatz 6 auf die Behälteröffnung 3 aufgesetzt und die Behälteröffnung 3 mit dem Deckel 4 verschlossen. Wenn nun die Mischvorrichtung 1 vom Anwender geschüttelt wird, gelangt der Folienbeutel 8 zwangsläufig mit dem zitruspressenartigen Einsatz 6 in Kontakt und wird dabei mechanisch beansprucht und zwar so stark, dass er, zumindest bereichsweise, aufreißt. Das pulverförmige Produkt 9 kann dann direkt aus dem Folienbeutel austreten und sich mit der Flüssigkeit vermischen, deren Füllhöhe in Ruhelage mit 10 angedeutet ist. Dadurch wird der Mischvorgang wesentlich beschleunigt, außerdem kann durch die Zerkleinerung des Folienbeutels 8 dieser auch schneller von der Flüssigkeit aufgelöst werden. Nach ausreichender Mischdauer, die von den zu mischenden Produkten abhängig ist, wird der Mischbehälter 2 wieder geöffnet, indem der Deckel 4 entfernt wird. Nachfolgend wird der Einsatz 6 entfernt und das fertige Produkt kann entnommen werden.

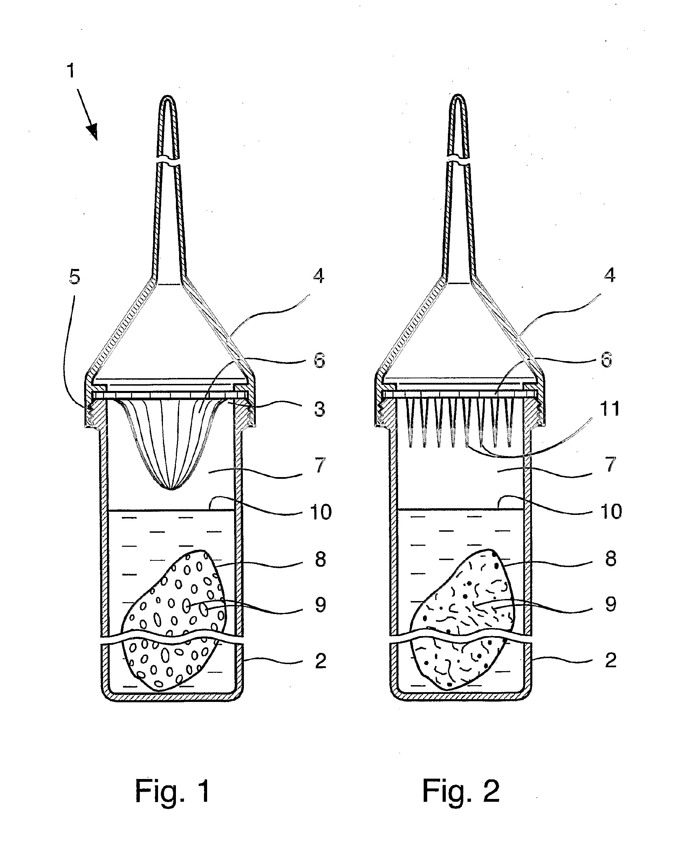

Die Ausführungsform nach Figur 2 unterscheidet sich von derjenigen nach Figur 1 nur durch einen anders gestalteten Einsatz 6'. Dieser Einsatz 6' ist siebplattenartig ausgebildet und mit in den Aufnahmeraum 7 gerichteten, spitz zulaufenden Stiften 11 versehen.
Beim Schütteln der Mischvorrichtung dringen die Stifte 11 wenigstens bereichsweise in den Folienbeutel 8 ein und führen zu dessen teilweiser Zerstörung, so dass das pulverförmige Produkt 9 leicht austreten und sich mit der Flüssigkeit vermischen kann.

Natürlich ist die Erfindung nicht auf die dargestellten Ausführungsbeispiele beschränkt. Weitere Ausgestaltungen sind möglich, ohne den Grundgedanken zu verlassen. So können anstelle der dargestellten Einsätze auch andere Einbauten zur mechanischen Einwirkung auf den Folienbeutel im Aufnahmeraum vorgesehen sein, diese können auch fest im Aufnahmeraum angeordnet sein.

Außerdem kann noch eine zusätzliche flüssigkeitsdichte Abdeckung oberhalb der Einsätze angebracht sein, die verhindert, dass während des Mischvorganges Flüssigkeit in den Raum oberhalb der Einsätze gelangt. Diese Abdeckung wird dann zusammen mit den Einsätzen nach Beendigung des Mischvorganges entfernt.

Alternativ ist vorzusehen, dass der Einsatz und der Deckel aus einem einzigen Element bestehen, welches zum Beispiel im Spritzgussverfahren hergestellt werden kann.

Als für den Benutzer einfachste Ausführung ist eine solche einteilige Ausführung noch mit einer als Dichtungsring ausgebildeten Abdichtung fest verbunden. Das für die Benutzung des Mischbehälters notwendige Verschließen erfordert in diesem einfachsten Fall nur einen einzigen Handgriff.

Darüber hinaus kann der Folienbeutel unter Überdruck stehen, was die Zerstörung durch die mechanische Belastung durch die Einbauten auch akustisch dem Benutzer mitteilt. Nach dem zu erwartenden Knall kann davon ausgegangen werden, dass der Beutel zumindest bereichsweise aufgerissen ist, und damit das enthaltene Produkt für den Mischvorgang bereit ist.

In einem weiteren vorteilhaften Fall ist das im Folienbeutel aufgenommene Produkt eine kosmetische Zubereitung und beides zusammen bildet eine kosmetische Portion.

### Beispiele für die Kosmetische Portion:

### Beispiel 1:

Es wurde eine erfindungsgemäße Portion, enthaltend ein Blondierpulver mit der in Tabelle 1 angegebenen Zusammensetzung, hergestellt.

**Tabelle 1: Blondierpulver**

| Rohstoff | Angabe in Gew.-% |
|---|---|
| Ammoniumpersulfat | 21,5 % |
| Natriumphosphat | 4,0 % |
| Aerosil 200 | 3,0 % |
| Kaliumpersulfat | 33,0 % |
| Britesil^{®} C 20 | 22,0 % |
| Natriumstearat | 8,0 % |
| Ceasit^{®}I | 4,0 % |
| Magnesiumoxid | 2,0 % |
| Magnesiumhydroxidcarbonat | 1,0 % |
| Lanette^{®} E | 1,0 % |
| Idranal^{®} III | 0,5 % |

Es wurden die folgenden Rohstoffe verwendet:

| | |
|---|---|
| Aerosil^{®} 200: | pyrogene Kieselsäure (INCl-Bezeichnung: Silica) (Degussa) |
| Britesil^{®} C20: | Natriumsilicat; Molverhältnis SiO₂:Na₂O= 2,0 |
| Ceasit^{®} I: | Calciumstearat |

| | |
|---|---|
| Lanette^{®}E: | Natriumcetylstearylsulfat (ex Cognis) |
| Idranal^{®} III: | Ethylendiamin-N,N,N',N'-tetraessigsäuredinatriumsalz |

Die Rohstoffkomponenten des Blondierpulvers werden vermischt und vermahlen, bis eine mittlere Teilchengröße von 100 µm eingestellt ist.

Das Blondierpulver wird nachfolgend mittels eines Schlauchbeutelsiegel-verfahrens in eine wasserlösliche PVA-Polymerfolie (Solublon, Typ SA 20 ex Syntana) verpackt.

Der Folienbeutel weist die folgenden Eigenschaften auf:

| | |
|---|---|
| quadratischer Mittelwert der Rauheit: | 30 µm |
| mittlere Stärke der Folie: | 20 µm |
| Folienmaterial: | teilverseiftes Polyvinylacetat mit einem Verseifungsgrad von 96 %; Gießfolie; mittleres Molekulargewicht: 36000 g/mol |

Außen- und Innenflächen der Polymerfolie sind dreidimensional strukturiert mit einem karoförmigen Muster. Das Muster wird gebildet durch ein Gitter mit quadratischen Vertiefungen, so dass die Gitternetzlinien durch die Ränder der Vertiefungen ausgebildet werden.

Die Tiefe der Vertiefung beträgt 0,12 mm.
Die aufgeprägten Karos weisen einen Durchmesser von 0,6 mm auf.

Die erfindungsgemäße Portion enthält 25 g des vorgenannten Blondierpulvers.

Die Portion wurde nachfolgend in einer Wasserstoffperoxiddispersion mit einer Zusammensetzung gemäß Tabelle 2 aufgelöst, bei 20 °C:

**Tabelle 2: Wasserstoffperoxiddispersion**

| Rohstoffe | Angaben in Gew.-% |
|---|---|
| Lorol^{®} C16 | 3,6 % |
| Eumulgin | 0,9 % |
| Texapon^{®} NSO | 2,25 % |
| Ammoniak (25 %) | 0,65 % |
| Dipicolinsäure | 0,1 % |
| Natriumpyrophosphat | 0,03 % |
| Turpinal^{®} SL | 1,5 % |
| Wasserstoffperoxid | 6 % |
| Wasser | ad 100) |

Es wurden die folgenden Rohstoffkomponenten eingesetzt:

| | |
|---|---|
| Lorol^{®} C16: | C₁₆-Fettalkohol |
| Eumulgin^{®}: | Ceteareth-20 |
| Texapon^{®} NSO: | Natrium-Lauryl-Ethersulfat mit 2 EO |
| Turpinal^{®} SL: | Hydroxethyldiphosphonsäure |

**Es zeigte sich, dass sich die erfindungsgemäße Portion ca. 5 mal so schnell auflöst wie die aus dem Stand der Technik bekannten Portionen mit Hüllmaterialien aus glatten wasserlöslichen Folien, vergleichbarer Stärken.**

Die erfindungsgemäße Portion befindet sich in einem Kit zusammen mit den folgenden Bestandteilen:
a) eine Mischvorrichtung
c) eine Kunststoffflasche mit einer Wasserstoffperoxiddispersion gemäß Tabelle 2
d) einen Conditioner

## Patentansprüche

1. Mischset mit einer Mischvorrichtung (1) zum Mischen eines in einem von einer Flüssigkeit löslichen Folienbeutel (8) aufgenommenen stückigen, gelartigen, pastösen, pulverförmigen oder flüssigen Produktes mit der Flüssigkeit und ggf. wenigstens einer weiteren Komponente, innerhalb eines verschließbaren Mischbehälters (2) mit einem Aufnahmeraum (7) für das in dem Folienbeutel (8) aufgenommene Produkt (9) und die Flüssigkeit und die ggf. weiteren Komponenten, **dadurch gekennzeichnet, dass** der Mischbehälter im Bereich des Aufnahmeraumes (7) Einbauten (6,6') zur mechanischen Zerstörung auf den Folienbeutel (8) aufweist, wobei das Mischset umfasst
a) einen von einer Flüssigkeit löslichen Folienbeutel (8), enthaltend ein Produkt (9), wie Pulver, Granulat, Paste, Gel, Flüssigkeit oder Tabletten, der an mindestens einer Oberfläche des Folienbeutels (8) eine dreidimensionale Struktur, vorzugsweise eine aufgeprägte dreidimensionale Struktur, aufweist, welche die dreidimensionale makroskopische Folienbeuteloberfläche gegenüber der zweidimensionalen geometrische Folienbeuteloberfläche vergrößert,
b) ggf. ein mit der Flüssigkeit gefülltes Aufnahmebehältnis sowie
c) ggf. ein mit einer weiteren Komponente gefülltes Aufnahmebehältnis.

2. Mischset nach Anspruch 1, **dadurch gekennzeichnet, dass** der Mischbehälter (2) eine von einem abnehmbaren Deckel (4), der vorzugsweise mittels einer Schraub-, Steck- oder Schnapp-Rastverbindung lösbar mit dem Mischbehälter verbunden ist, verschließbare Behälteröffnung (3) aufweist.

3. Mischset nach Anspruch 2, **dadurch gekennzeichnet, dass** ein die Einbauten (6, 6') aufweisender Einsatz (6,6') und der Deckel (4) fest miteinander verbunden sind.

4. Mischset nach Anspruch 3, **dadurch gekennzeichnet, dass** der Einsatz (6) zum Aufnahmeraum (7) hin zitruspressenartig ausgewölbt ist.

5. Mischset nach Anspruch 3, **dadurch gekennzeichnet, dass** die Einbauten (6') siebplattenartig mit mindestens einem oder mehreren, in den Aufnahmeraum (7) gerichteten, spitz zulaufenden Stiften (11) oder Dornen ausgebildet sind.

6. Mischset nach Anspruch 3, **dadurch gekennzeichnet, dass** die Einbauten (6') in den Aufnahmeraum (7) gerichtete, messerartige Elemente aufweisen.

7. Mischset nach einem der vorhergehenden Ansprüche, **dadurch gekennzeichnet, dass** der Innendruck des Folienbeutels (8) größer als der Umgebungsdruck ist.

8. Mischset nach einem der vorangegangenen Ansprüche **dadurch gekennzeichnet, dass** die Oberfläche des Folienbeutels (8) einen quadratischen Mittelwert der Rauheit von wenigstens 10 µm aufweist, insbesondere von 10 bis 100 µm, vorzugsweise 10 bis 50 µm, besonders bevorzugt von 30 bis 35 µm.

9. Mischset nach einem der vorangegangenen Ansprüche, **dadurch gekennzeichnet, dass** die dreidimensionale makroskopische Oberfläche des Folienbeutels (8) mindestens 10%, bevorzugt mindestens 20 %, weiter bevorzugt zwischen 20% und 100%, äußerst bevorzugt zwischen 30% und 50 % größer als die zweidimensionale geometrische Oberfläche ist.

10. Mischset nach einem der vorangegangenen Ansprüche, **dadurch gekennzeichnet, dass** die Außen- und/oder Innenoberfläche des Folienbeutels (8) zu mindestens 50 %, vorzugsweise mindestens zu 70 %, weiter bevorzugt mindestens zu 90 % und insbesondere im Wesentlichen vollständig mit einer dreidimensionalen, vorzugsweise aufgeprägten, Struktur versehen ist.

11. Mischset nach einem der vorangegangenen Ansprüche, **dadurch gekennzeichnet, dass** der Folienbeutel (8) eine aufgeprägte dreidimensionale Struktur in Form eines regelmäßigen Musters aufweist.

12. Mischset nach Anspruch 11, **dadurch gekennzeichnet, dass** das regelmäßige Muster in einer periodisch wiederkehrenden Anordnung von Erhöhungen und

13. Mischset nach Anspruch 11 oder 12, **dadurch gekennzeichnet, dass** das aufgeprägte Muster mindestens 4, vorzugsweise mindestens 6, besonders bevorzugt zwischen 8 und 50, weiter bevorzugt zwischen 10 und 25 Vertiefungen oder Erhöhungen pro 1 cm² der zweidimensionalen Oberfläche aufweist.

14. Mischset nach einem der vorangegangenen Ansprüche, **dadurch gekennzeichnet, dass** die Oberfläche des Folienbeutels (8) mit einem gitterförmigen oder wabenförmigen dreidimensionalem strukturierten Muster versehen ist.

15. Mischset nach Anspruch 14, **dadurch gekennzeichnet, dass** das Verhältnis der durchschnittlichen Breite von Gitternetzlinie zur maximalen Ausdehnung in der Ebene der Vertiefung kleiner 20:1, vorzugsweise kleiner 10:1, besonders bevorzugt kleiner 1:1, weiter bevorzugt kleiner 0,5:1 und insbesondere kleiner 0,25:1 ist.

16. Mischset nach Anspruch 15, **dadurch gekennzeichnet, dass** das Verhältnis von durchschnittlichem Durchmesser der Vertiefung zur Tiefe der Vertiefung unterhalb 20:1, vorzugsweise 10:1 bis 1:10, insbesondere 8:1 bis 1:1, im Speziellen 6:1 bis 4:1 beträgt.

17. Mischset nach einem der vorangegangenen Ansprüche, **dadurch gekennzeichnet, dass** die mittlere Stärke des Folienbeutels (8) 10 bis 100 µm, vorzugsweise 15 bis 50 µm und insbesondere 20 bis 40 µm beträgt.

18. Mischset nach einem der vorangegangenen Ansprüche, **dadurch gekennzeichnet, dass** der Folienbeutel (8) ganz oder teilweise besteht aus einem Thermoplasten, der ausgewählt ist aus der Gruppe bestehend aus Polyvinylalkohol (PVA), acetalisiertem Polyvinylalkohol, Polyvinylpyrrolidon, Polyethylenoxid, Gelatine, Cellulose und Stärke, und/oder Mischungen der vorgenannten Polymere, wobei Polyvinylalkohol besonders bevorzugt ist.

19. Mischset nach einem der vorangegangenen Ansprüche, **dadurch gekennzeichnet, dass** der Folienbeutel (8) zusätzlich Polymere umfasst, die ausgewählt sind aus der Gruppe bestehend aus Acrylsäure-haltigen Polymeren, Polyacrylamiden, Oxazolin- Polymeren, Polystyrolsulfonaten, Polyurethanen, Polyestern, Polyethern und/oder Mischungen der vorgenannten Polymere.

20. Mischset nach einem der vorangegangenen Ansprüche, **dadurch gekennzeichnet, dass** der Folienbeutel (8) ein acetalisiertes Polyvinylalkohol mit einem Verseifungsgrad 70 bis 100 Mol-%, vorzugsweise von 80 bis 96 Mol%, besonders bevorzugt von 82 bis 94 Mol-% und insbesondere von 85 bis 89 Mol-% umfasst.

21. Mischset nach einem der vorangegangenen Ansprüche, **dadurch gekennzeichnet, dass** der Folienbeutel (8) ein Innenvolumen von 5 bis 500 cm³, vorzugsweise von 10 bis 200 cm³, besonders bevorzugt von 20 bis 100 cm³, insbesondere von 30 bis 70 cm³ aufweist.

22. Mischset nach einem der vorangegangenen Ansprüche, **dadurch gekennzeichnet, dass** sich der Folienbeutel (8) in Wasser bei 20 °C innerhalb von weniger als 5 Minuten, vorzugsweise weniger als 4 Minuten, weiter bevorzugt weniger als 3 Minuten, insbesondere zwischen 2 und 0,5 Minuten vollständig auflöst.

23. Mischset nach einem der vorangegangenen Ansprüche, **dadurch gekennzeichnet, dass** der Folienbeutel (8) mehrere Kammern aufweist.

## Claims

1. Mixing set comprising a mixing apparatus (1) for mixing a lumpy, gelatinous, pasty, powdery or liquid product, received in a film bag (8) soluble in a liquid, with the liquid and optionally at least one further component, within a closable mixing container (2) having a reception space (7) for the product (9) received in the film bag (8) and the liquid and the optionally present further components, **characterised in that** the mixing container includes, in the area of the reception space (7), mountings (6, 6') for mechanically disrupting the film bag (8), wherein the mixing set comprises
a. a film bag (8) soluble in a liquid and containing a product (9) such as powder, granulate, paste, gel, liquid or tablets, which film bag on at least one surface of the film bag (8) has a three-dimensional structure, preferably an embossed three-dimensional structure, which enlarges the three-dimensional macroscopic film bag surface relative to the two-dimensional geometric film bag surface,
b. optionally a reception container filled with the liquid, as well as
c. optionally a reception container filled with a further component.

2. Mixing set as claimed in claim 1, **characterised in that** the mixing container (2) has a container opening (3) that can be closed with a removable lid (4) which is preferably releasably connected to the mixing container by means of a screw, plug-in or snap-on connection.

3. Mixing set as claimed in claim 2, **characterised in that** an insert (6, 6') including the mountings (6, 6') and the lid (4) are firmly connected to each other.

4. Mixing set as claimed in claim 3, **characterised in that** the insert (6) is convexly curved like a lemon press towards the reception space (7).

5. Mixing set as claimed in claim 3, **characterised in that** the mountings (6') are formed like a sieve plate with at least one or more tapered pins (11) or spikes orientated into the reception space (7).

6. Mixing set as claimed in claim 3, **characterised in that** the mountings (6') include knife-like elements orientated into the reception space (7).

7. Mixing set as claimed in any one of the preceding claims, **characterised in that** the internal pressure of the film bag (8) is greater than the ambient pressure.

8. Mixing set as claimed in any one of the preceding claims, **characterised in that** the surface of the film bag (8) has a root mean square value of roughness of at least 10 µm, in particular of 10 to 100 µm, preferably of 10 to 50 µm, particularly preferably of 30 to 35 µm.

9. Mixing set as claimed in any one of the preceding claims, **characterised in that** the three-dimensional macroscopic surface of the film bag (8) is at least 10%, preferably at least 20%, more preferably between 20% and 100%, extremely preferably between 30% and 50% greater than the two-dimensional geometrical surface.

10. Mixing set as claimed in any one of the preceding claims, **characterised in that** at least 50%, preferably at least 70%, more preferably at least 90% and in particular substantially all of the external and/or internal surface of the film bag (8) is provided with a three-dimensional, preferably embossed, structure.

11. Mixing set as claimed in any one of the preceding claims, **characterised in that** the film bag (8) has an embossed three-dimensional structure in the form of a regular pattern.

12. Mixing set as claimed in claim 11, **characterised in that** the regular pattern consists of a periodically recurring arrangement of elevations and indentations of the film bag surface.

13. Mixing set as claimed in claim 11 or 12, **characterised in that** the embossed pattern has at least 4, preferably at least 6, particularly preferably between 8 and 50, more preferably between 10 and 25, indentations or elevations per 1 cm² of the two-dimensional surface.

14. Mixing set as claimed in any one of the preceding claims, **characterised in that** the surface of the film bag (8) is provided with a grid-shaped or honeycomb-shaped three-dimensional structured pattern.

15. Mixing set as claimed in claim 14, **characterised in that** the ratio between the average width of the gridline and the maximum extension at the level of the indentation is less than 20:1, preferably less than 10:1, particularly preferably less than 1:1, more preferably less than 0.5:1 and in particular smaller than 0.25:1.

16. Mixing set as claimed in claim 15, **characterised in that** the ratio between the average diameter of the indentation and the depth of the indentation is less than 20:1, preferably 10:1 to 1:10, in particular 8:1 to 1:1, especially 6:1 to 4:1.

17. Mixing set as claimed in any one of the preceding claims, **characterised in that** the medium strength of the film bag (8) is 10 to 100 µm, preferably 15 to 50 µm and in particular 20 to 40 µm.

18. Mixing set as claimed in any one of the preceding claims, **characterised in that** the film bag (8) completely or partially consists of a thermoplast selected from the group consisting of polyvinyl alcohol (PVA), acetalised polyvinyl alcohol, polyvinyl pyrrolidone, polyethylene oxide, gelatine, cellulose and starch, and/or mixtures of the above-mentioned polymers, of which polyvinyl alcohol is particularly preferred.

19. Mixing set as claimed in any one of the preceding claims, **characterised in that** the film bag (8) additionally comprises polymers selected from the group consisting of acrylic acid-containing polymers, polyacrylamides, oxazoline polymers, polystyrene sulfonates, polyurethanes, polyesters, polyethers and/or mixtures of the above-mentioned polymers.

20. Mixing set as claimed in any one of the preceding claims, **characterised in that** the film bag (8) comprises an acetalised polyvinyl alcohol having a degree of saponification of 70 to 100 mol %, preferably of 80 to 96 mol %, particularly preferably of 82 to 94 mol %, and in particular of 85 to 89 mol %.

21. Mixing set as claimed in any one of the preceding claims, **characterised in that** the film bag (8) has an internal volume of 5 to 500 cm³, preferably of 10 to 200 cm³, particularly preferably of 20 to 100 cm³, in particular of 30 to 70 cm³.

22. Mixing set as claimed in any one of the preceding claims, **characterised in that** the film bag (8) completely dissolves in water at 20°C within less than 5 minutes, preferably less than 4 minutes, more preferably less than 3 minutes, in particular between 2 and 0.5 minutes.

23. Mixing set as claimed in any one of the preceding claims, **characterised in that** the film bag (8) includes a plurality of chambers.

## Revendications

1. Ensemble de mélange comportant un dispositif de mélange (1) servant à mélanger un produit, se présentant sous la forme d'un liquide, d'une poudre, d'une pâte, d'un gel ou de morceaux, qui est placé dans un sachet de film (8) soluble dans un liquide, avec le liquide et éventuellement au moins un autre composant, à l'intérieur d'un récipient de mélange (2), pouvant être fermé, qui comporte un espace de réception (7) destiné au produit (9), placé dans le sachet de film (8), et au liquide et éventuellement aux autres composants, **caractérisé en ce que** le récipient de mélange comporte au niveau de l'espace de réception (7) des éléments encastrés (6, 6') destinés à la destruction mécanique du sachet de film (8), l'ensemble de mélange comportant
a) un sachet de film (8), soluble dans un liquide, contenant un produit (9) tel qu'une poudre, un granulat, une pâte, un gel, un liquide ou des comprimés, qui possède au niveau d'au moins une surface du sachet de film (8) une structure tridimensionnelle, de préférence une structure tridimensionnelle imprimée, qui augmente la surface macroscopique tridimensionnelle du sachet de film par rapport à la surface géométrique bidimensionnelle du sachet de film,
b) éventuellement un récipient rempli avec le liquide et
c) éventuellement un récipient rempli avec un autre composant.

2. Ensemble de mélange selon la revendication 1, **caractérisé en ce que** le récipient de mélange (2) comporte une ouverture de récipient (3) qui peut être fermée par un couvercle amovible (4) qui est relié de préférence par une liaison par vissage, enfichage ou encliquetage de façon amovible au récipient de mélange.

3. Ensemble de mélange selon la revendication 2, **caractérisé en ce qu'**un insert (6, 6'), comportant les éléments encastrés (6, 6'), et le couvercle (4) sont reliés solidairement entre eux.

4. Ensemble de mélange selon la revendication 3, **caractérisé en ce que** l'insert (6) est bombé à la manière d'un presse-agrumes en direction de l'espace de réception (7).

5. Ensemble de mélange selon la revendication 3, **caractérisé en ce que** les éléments encastrés (6') sont configurés sous la forme d'une plaque de tamis comportant au moins un ou plusieurs picots (11) ou goujons s'étendant en pointe et dirigés dans l'espace de réception (7).

6. Ensemble de mélange selon la revendication 3, **caractérisé en ce que** les éléments encastrés (6') comportent des éléments en forme de couteaux dirigés dans l'espace de réception (7).

7. Ensemble de mélange selon l'une des revendications précédentes, **caractérisé en ce que** la pression interne dans le sachet de film (8) est supérieure à la pression ambiante.

8. Ensemble de mélange selon l'une des revendications précédentes, **caractérisé en ce que** la surface du sachet de film (8) a une rugosité moyenne quadratique d'au moins 10 µm, en particulier de 10 à 100 µm, de préférence 10 à 50 µm, plus préférablement de 30 à 35 µm.

9. Ensemble de mélange selon l'une des revendications précédentes, **caractérisé en ce que** la surface macroscopique tridimensionnelle du sachet de film (8) est supérieure à la surface géométrique bidimensionnelle d'au moins 10%, de préférence d'au moins 20%, plus préférablement d'une valeur comprise entre 20% et 100%, de manière préférée entre 30% et 50%.

10. Ensemble de mélange selon l'une des revendications précédentes, **caractérisé en ce que** la surface extérieure et/ou intérieure du sachet de film (8) est à 50% au moins, de préférence 70% au moins, plus préférablement 90% au moins et notamment sensiblement entièrement pourvue d'une structure tridimensionnelle, de préférence imprimée.

11. Ensemble de mélange selon l'une des revendications précédentes, **caractérisé en ce que** le sachet de film (8) a une structure tridimensionnelle imprimée se présentant sous la forme d'un motif régulier.

12. Ensemble de mélange selon la revendication 11, **caractérisé en ce que** le motif régulier consiste en un agencement, se répétant périodiquement, de saillies et de creux à la surface du sachet de film.

13. Ensemble de mélange selon la revendication 11 ou 12, **caractérisé en ce que** le motif imprimé comporte au moins 4, de préférence au moins 6, plus préférablement entre 8 et 50, plus préférablement encore entre 10 et 25, creux ou saillies par cm² de la surface bidimensionnelle.

14. Ensemble de mélange selon l'une des revendications précédentes, **caractérisé en ce que** la surface du sachet de film (8) est pourvue d'un motif structuré tridimensionnel en forme de grille ou en nid d'abeilles.

15. Ensemble de mélange selon la revendication 14, **caractérisé en ce que** le rapport de la largeur moyenne de la ligne de grille sur l'extension maximale dans le plan du creux est inférieur à 20:1, de préférence inférieur à 10:1, plus préférablement inférieur à 1:1, encore plus préférablement inférieur à 0,5:1 et en particulier inférieur à 0,25:1.

16. Ensemble de mélange selon la revendication 15, **caractérisé en ce que** le rapport du diamètre moyen du creux sur la profondeur du creux est inférieur à 20:1, de préférence de 10:1 à 1:10, en particulier de 8:1 à 1:1, spécialement de 6:1 à 4:1.

17. Ensemble de mélange selon l'une des revendications précédentes, **caractérisé en ce que** l'épaisseur moyenne du sachet de film (8) est de 10 à 100 µm, de préférence de 15 à 50 µm et en particulier de 20 à 40 µm.

18. Ensemble de mélange selon l'une des revendications précédentes, **caractérisé en ce que** le sachet de film (8) est totalement ou partiellement constitué d'une matière thermoplastique qui est choisie dans le groupe constitué par l'alcool polyvinylique (PVA), l'alcool polyvinylique acétalisé, la polyvinylpyrrolidone, le poly(oxyde d'éthylène), la gélatine, la cellulose et l'amidon et/ou des mélanges des polymères susmentionnés, l'alcool polyvinylique étant particulièrement préféré.

19. Ensemble de mélange selon l'une des revendications précédentes, **caractérisé en ce que** le sachet de film (8) comprend en outre des polymères qui sont choisis dans le groupe constitué par des polymères contenant de l'acide acrylique, des polyacrylamides, les polymères d'oxazoline, les polystyrène sulfonates, les polyuréthanes, les polyesters, les polyéthers et/ou les mélanges des polymères susmentionnés.

20. Ensemble de mélange selon l'une des revendications précédentes, **caractérisé en ce que** le sachet de film (8) comporte un alcool polyvinylique acétalisé ayant un degré de saponification de 70 à 100% en moles, de préférence de 80 à 96% en moles, plus préférablement de 82 à 94% en moles, et en particulier de 85 89% en moles.

21. Ensemble de mélange selon l'une des revendications précédentes, **caractérisé en ce que** le sachet en film (8) a un volume intérieur de 5 à 500 cm³, de préférence de 10 à 200 cm³, plus préférablement de 20 à 100 cm³, en particulier de 30 à 70 cm³.

22. Ensemble de mélange selon l'une des revendications précédentes, **caractérisé en ce que** le sachet de film (8) se dissout complètement dans de l'eau à 20°C en moins de 5 minutes, de préférence en moins de 4 minutes, plus préférablement en moins de 3 minutes, en particulier entre 2 et 0,5 minutes.

23. Ensemble de mélange selon l'une des revendications précédentes, **caractérisé en ce que** le sachet de film (8) comporte une pluralité de chambres.
